# EUROPÄISCHE PATENTSCHRIFT

(11) **EP 4 097 402 B1**
(45) Veröffentlichungstag und Bekanntmachung des Hinweises auf die Patenterteilung: **24.04.2024**
(21) Anmeldenummer: 20793577.6
(22) Anmeldetag: 07.10.2020
(51) Int. Cl.: F24F 3/163, A61G 10/00, E04H 15/20, F24F 3/167, F24F 13/02, F24F 13/068, F24F 11/00

(54) **VORRICHTUNG MIT EINER SCHUTZHÜLLE UND DEREN VERWENDUNG**
DEVICE WITH A PROTECTIVE COVER AND IT'S USE
DISPOSITIF AVEC UNE GAINE PROTECTIVE ET SON USAGE

(30) Priorität: 28.01.2020 DE 102020200982; 16.04.2020 DE 102020110451
(43) Veröffentlichungstag der Anmeldung: 07.12.2022
(73) Patentinhaber: Fraunhofer-Gesellschaft zur Förderung der angewandten Forschung e.V., 80686 München (DE)
(72) Erfinder: GOMMEL, Udo, 70569 Stuttgart (DE); BÜRGER, Frank, 70569 Stuttgart (DE); FALCH, Daniel, 70569 Stuttgart (DE); KOPCOK, Miroslav, 70569 Stuttgart (DE); KELLER, Markus, 70569 Stuttgart (DE); HOFFMANN, Viola, 70569 Stuttgart (DE)
(74) Vertreter: Rösler, Uwe
(86) Internationale Anmeldenummer: PCT/EP2020/078107
(87) Internationale Veröffentlichungsnummer: WO 2021/151531

(56) Entgegenhaltungen:
- EP-A1- 0 859 105
- WO-A1-2007/102798
- DE-A1- 2 523 512
- US-A- 3 936 984
- US-A- 4 581 986

## Beschreibung

### Technisches Gebiet

Die Erfindung bezieht sich auf eine Vorrichtung mit einer Schutzhülle, die dafür geeignet und ausgebildet ist, wenigstens einen Raumbereich zu umhüllen, so dass die Schutzhülle den wenigstens einen Raumbereich gegenüber einer die Schutzhülle unmittelbar umgebenden Umgebung zu separieren vermag, sowie deren Verwendung. Zudem werden Verfahren zum Betreiben einer produktionstechnischen Einheit unter Reinraumbedingungen sowie eines von der Schutzhülle umgebenden viren- und querkontaminationsfreien Raumbereiches zur Unterbringung quarantänepflichtiger Personen und/oder Objekte beschrieben.

### Stand der Technik

Üblicherweise stellen Rein- und Reinsträume komplex und technisch aufwendig gestaltete Areale dar, deren Zugang zumeist über unterschiedliche Schleusensysteme realisiert wird. Reinräume werden mit speziell konfektionierten klimatechnischen Aggregaten gespeist, die sicherstellen, dass Verunreinigungen sofort aus der Luft entfernt werden.

Für den Betrieb eines Reinraumes werden zu Klassifizierung und Qualitätsüberprüfung Partikelmessungen durchgeführt, durch die eine Klassifizierung der Reinheit des Raumes möglich wird. So ist in der deutschen Industrienorm DIN EN ISO 14644-1:2016-06 eine Unterteilung in neun unterschiedliche Reinheitsklassen ISO 1 bis ISO 9 vorgesehen. Entsprechende Reinraumqualitätsanforderungen in Bezug auf lebensmittelrelevante Mikroorganismen pro m³ Luft sind in der Richtlinie VDI 2083 geregelt. Eine für pharmazeutische Reinraumanwendungen relevante normierte Anzahl keimbildender Einheiten KBE ist in der Klassifizierung nach dem EU-GMT Leitfaden Annex 1 geregelt.

Es zeigen die jüngsten Ereignisse i.V.m. epidemiologischen Virenausbreitungen, dass im Falle von zu befürchtenden Pandemien potenziell infizierte Personen von nichtinfizierten Personen schnellstmöglich getrennt werden müssen. Da sich bei der Ausbreitung virenbasierter Seuchen oft ein exponentieller Anstieg der Fallzahlen zeigt, müssen innerhalb kürzester Zeiträume möglichst viele Kapazitäten für eine räumliche Isolierung, d.h. Quarantänebereiche, zur Verfügung gestellt werden. Ein Vorhalten von festinstallierten Quarantänebereichen, z.B. kompletter Krankenhäuser, ist aufgrund der geographisch-räumlichen Varianz, zumeist nicht möglich. Die Ausbreitung von Seuchen muss direkt am Entstehungsort, der an beliebigen Stellen der Erde auftreten kann, bekämpft werden. Somit müssen schnell aufzubauende und zu installierende Systeme mit einer hohen Wirksamkeit der Verhinderung der Biokontaminationsverschleppung zum Einsatz kommen.

Das Problem wurde bislang nur unzureichend gelöst, da bisherige Systeme
- meist festinstallierte Quarantänestationen sind,
- diese Quarantänestationen meist nicht am richtige Ort der Welt aufgebaut wurden bzw. verfügbar sind,
- die benötigte Anzahl der schnell transportierbaren und aufbaubaren Quarantänesysteme nicht gegeben ist.

Zudem können Krankenhäuser bislang keine ausreichende Zahl von Quarantänezimmern zur Verfügung stellen. Sofern in offenen Hallen eine Unterbringung einer Vielzahl von potenziell infizierten Personen erfolgt, ist die Wahrscheinlichkeit der Übertragung auf die restlichen, bis dato gesunden Personen, vorprogrammiert.

Die Druckschrift DE 25 23 512 A1 offenbart eine Hallenkonstruktion, die eine bspw. aus Gewebe bestehende Hallenaußenwand sowie Halleninnenwand vorsieht, die beide über Abstandshalter voneinander beabstandet sind. In dem Zwischenraumbereich zwischen beiden Hallenwände erfolgt eine Zuströmung mit Hilfe einer Strömungseinheit, die zur Erzeugung rein mechanischer Tragkräfte für einen genügend großen Überdruck innerhalb des Zwischenraumbereiches sorgt. Damit offenbart die Druckschrift DE 25 23 512 A1 eine Vorrichtung mit Schutzhülle gemäß dem Oberbegriff von Anspruch 1.

Die Druckschrift WO 2007/102798 A1 offenbart eine Zeltanordnung zum Zwecke einer Dekontamination, die über eine aufblasbare Struktur verfügt. Auch in diesem Fall verfügt die Zeltanordnung über eine aufblasbare Struktur mit einer Außen- und Innenwand, die über Abstandshaltesegmente unter Ausbildung eines durchströmbaren Zwischenraumes beabstandet sind und mit einer Strömungseinheit zur Erzeugung mechanischer Stützkräfte befüllt wird.

### Darstellung der Erfindung

Der Erfindung liegt die Aufgabe zugrunde, eine Vorrichtung mit einer Schutzhülle, die dafür geeignet und ausgebildet ist, wenigstens einen Raumbereich derart zu umhüllen, so dass die Schutzhülle den wenigstens einen Raumbereich gegenüber einer die Schutzhülle unmittelbar umgebenden Umgebung zu separieren vermag, derart weiterzubilden, so dass die Schutzhülle den hohen Standards an Reinheitsanforderungen entspricht, wie sie in der deutschen Industrienorm DIN EN ISO 14644-1:2016-06 gefordert werden. Die lösungsgemäße Vorrichtung mit Schutzhülle soll die vorstehend zum Stand der Technik erläuterten Nachteile vermeiden und einen schnellen Einsatz an einem beliebigen Ort ermöglichen. So soll die Vorrichtung dafür geeignet sein, schnell und flexibel an beliebigen Orten installiert werden zu können und die Möglichkeit zur Einrichtung von viren- und querkontaminationsfreien Raumbereichen zu schaffen, die für eine sichere Unterbringung quarantänepflichtiger Personen und/oder Objekte geeignet sein soll.

Zur Lösung der obigen Aufgabe wird erfindungsgemäß eine Vorrichtung mit einer Schutzhülle gemäß Anspruch 1 vorgeschlagen. Bevorzugte Ausführungsformen der Erfindung sind in den Ansprüchen 2-12 definiert.

Gegenstand des Anspruches 13 betrifft eine bevorzugte Verwendung der Vorrichtung.

Die lösungsgemäße Vorrichtung mit einer Schutzhülle, die dafür geeignet und ausgebildet ist, wenigstens einen Raumbereich zu umhüllen, so dass die Schutzhülle den Raumbereich gegenüber einer die Schutzhülle unmittelbar umgebenden Umgebung zu separieren vermag, wobei die Schutzhülle wenigstens zwei, eine erste und eine zweite, durch eine Abstandschicht getrennte Schichten aufweist, von denen wenigstens die erste Schicht aus einem strömungsdurchlässigen Material gefertigt ist. Die Abstandsschicht weist eine die beiden Schichten auf Abstand haltende und in Schichtlängserstreckung durchströmbare, d.h. Gas-durchströmbare, Tragstruktur auf, die vorzugsweise in Form eines Abstandsgewirkes ausgebildet ist. Ferner ist wenigstens eine Strömungseinheit mit einem Strömungsausgang vorgesehen, der durch die der Umgebung zugewandte, zweite Schicht der Schutzhülle fluidisch mit der Abstandsschicht verbunden ist.

Die erste und zweite Schicht besteht jeweils aus textilem Material, wobei die erste Schicht über eine Strömungsdurchlässigkeit verfügt, die im Bereich von 500 bis 49000 m³/m²/h liegt. Demgegenüber ist die zweite Schicht, insbesondere zum Zwecke der Umhüllung von Produktions- und Automatisierungskomponenten strömungsundurchlässig bzw. weitgehend strömungsundurchlässig, d.h. sie besitzt eine Strömungsdurchlässigkeit von 0 bis 100 m³/m²/h. Im Falle der Verwendung der Schutzhülle als Quarantänemaßnahme ist die zweite Schicht ebenfalls Strömungsdurchlässigkeit, verfügt jedoch maximal über die gleiche Strömungsdurchlässigkeit wie die erste Schicht, vorzugsweise über eine geringere Strömungsdurchlässigkeit als die erste Schicht.

Die Schutzhülle wird relativ zum Raumbereich derart angebracht, so dass die strömungsdurchlässige erste Schicht dem Raumbereich zugewandt orientiert ist. Zumindest die erste und zweite Schicht sind ausschließlich aus reinraumtauglichen Material gefertigt, das dazu geeignet ist, einen Reinraum der ISO-Klassen 1 bis 9 nach DIN EN ISO 14644-1 :2016-06 unmittelbar zu begrenzen. Zudem weist die Schutzhülle einen Auslassbereich auf, durch den Überdruck-getrieben Raumluft aus dem Raumbereich in die Umgebung austritt und an dem eine Sterilisationseinheit angebracht ist. Schließlich ist die wenigstens eine Strömungseinheit derart betreibbar, dass ein sich innerhalb der Abstandsschicht ausbildender Schichtinnendruck sowohl größer als ein sich innerhalb des Raumbereiches ausbildender Rauminnendruck p1 sowie größer als ein in der Umgebung vorherrschender Umgebungsdruck p3 ist.

Mit Hilfe einer weiteren Strömungseinheit, vorzugsweise in Form einer Filter-Ventilator-Einheit, die zur Herstellung von Reinstluft aus der Umgebungsluft geeignet ausgebildet ist, wird Reinstluft in den Raumbereich, bspw. zwischen der Schutzhülle und der innerhalb des Raumbereiches angeordneten Einheit eingespeist. Alternativ ist es möglich, anstelle von Reinstluft auch ein technisches Gas in den Raumbereich einzuspeisen. Hierzu ist die Strömungseinheit mit einem technischen Gasreservoir verbunden.

Durch Einspeisen von Reinstluft bzw. eines technischen Gases, bspw. Wasserstoffperoxid (H₂O₂), in den Raumbereich bildet sich eine Druckdifferenz zwischen dem Raumbereich und der Abstandsschicht innerhalb der Schutzhülle aus, wodurch sich eine Stoffströmung aus dem Raumbereich durch die der Einheit zugewandten ersten, strömungsdurchlässigen Schicht in die Abstandsschicht etabliert. Die mit möglichen Partikel- und/oder Molekülkontaminationen versetzte Stoffströmung wird aus der Abstandsschicht über eine entsprechende fluidische Abführung bzw. Ableitung entsorgt oder entsprechend aufgereinigt. Da die der Umgebung, d.h. dem Reinraumbereich, zugewandte zweite Schicht der Schutzhülle strömungsundurchlässig ausgebildet ist, gelangen keinerlei Kontaminationen jeglicher Art aus der Abstandsschicht in die Umgebung.

Die Schutzhülle, die in einer einfachsten Ausführungsform aus zwei über eine Abstandsschicht voneinander beabstandete textile Schichten mit jeweils unterschiedlichen Strömungsdurchlässigkeiten besteht, ist vorzugsweise aus flexiblen und elastischen, kontaminationsfreien Textilmaterial gefertigt, um so die Beweglichkeit der produktionstechnischen Einheit nicht zu beeinträchtigen.

Eine weitere Ausführungsform sieht eine dritte und eine vierte, jeweils durch eine weitere Abstandsschicht voneinander getrennte Schichten vor, von denen die Dritte und die vorstehend erläuterte zweite Schicht, über eine Fügung miteinander verbunden oder einstückig, d.h. monolithisch gefertigt sind. Die jeweils dritte und zweite Schicht sind aus einem strömungsundurchlässigen Material gefertigt, d. h. das diesbezügliche textile Schichtmaterial weist eine Durchlässigkeit von lediglich 0 bis 100 m³/m²/h auf. Die vierte Schicht hingegen ist strömungsdurchlässig ausgebildet und verfügt über eine Strömungsdurchlässigkeit im Bereich zwischen 500 bis 49000 m³/m²/h. Die Strömungsdurchlässigkeiten der jeweils ersten und vierten Schicht müssen nicht notwendigerweise identisch sein und können voneinander abweichen. Gleichsam der Abstandsschicht im vorstehend erläuterten Ausführungsbeispiel, weist auch die weitere Abstandsschicht eine in Schichtlängserstreckung Gas-durchströmbare Tragstruktur auf, vorzugsweise in Form eines Abstandgewirkes.

Denkbar sind auch weiterführende Multilayerkombinationen, bei denen jeweils zwei der vorstehend erläuterten Schichtsysteme, jeweils umfassend die erste bis vierte Schicht mit den beiden dazwischenliegend angeordneten Abstandsschichten, über eine ihre jeweils strömungsdurchlässigen Schichten kombiniert sind. Die Multilayerkombinationen können auf diese Weise beliebig bezüglich ihrer Schichtanzahl skaliert werden.

Als Strömungseinheiten zur Strömungsbeaufschlagung in die Schutzhülle bzw. in den die produktionstechnische Einheit enthaltenen Raumbereich eignen sich vorzugsweise sog. Filter-Ventilator-Einheiten, die mit Überdruck betreibbar sind.

Überdies sind Filter-Ventilator-Einheiten in der Lage Reinstluft durch Ansaugen von Umgebungsluft durch eine entsprechende Filterwahl zu erzeugen.

In einer weiteren Ausführungsform sieht die Schutzhülle an oder innerhalb der einzelnen Schichten wenigstens einen Sensor vor, der Zustandsgrößen innerhalb der lösungsgemäß ausgebildeten Schutzhülle zu erfassen vermag. Hierzu eignen sich Sensoren der nachfolgenden Art: Sensor für partikuläre Kontaminationen (PAC), Sensor für molekulare oder chemische Kontaminationen (MOC), Temperatursensor (T), Drucksensor (p), Feuchtesensor (h), Strömungssensor, elektrostatischer Sensor (ESD) sowie Sensor für Biokontaminationen.

Die in den jeweiligen Schichten bzw. zwischen den Schichten angebrachten Sensoren werden mit an sich bekannten steifen oder elastisch-flexiblen, elektrischen Leiterstrukturen zur elektrischen Energieversorgung sowie Signalzu- und -abführung elektrisch kontaktiert, durch die die elastischen Eigenschaften der Schutzhülle nicht oder nur unwesentlich beeinträchtigt werden.

Die mit Hilfe der Sensoren erfassten Signale werden einer vorzugsweise separat zur Schutzhülle angeordneten Auswerte- und Steuereinheit zugeführt, in der die Sensorsignale in geeigneter Form ausgewertet und geeigneten Kenngrößen zugeordnet werden. Mit Hilfe der Sensoren sind die Erfassung von Kontaminationsgrößen, wie bspw. Partikelanzahl, Partikelgröße, Grad der chemischen Verunreinigung etc. sowie Kenngrößen über die Strömungsverhältnisse innerhalb des die Einheit umfassenden Raumbereiches sowie innerhalb der jeweiligen Abstandsschicht bestimmbar. Darüber hinaus können auch Verformungen innerhalb der Schutzhülle sowie auch mögliche Materialüberbeanspruchungen, die bis hin zum Materialversagen führen können, erfasst werden. Alle erfassbaren Sensorsignale dienen in vorteilhafter Weise zur Regelung bzw. Steuerung der wenigstens einen aktiv mit der Schutzhülle in fluidischer Wirkverbindung stehenden Strömungsmaschine. Die Steuerung bzw. Regelung der in den jeweiligen Abstandsschichten bzw. dem Raumbereich vorherrschenden Druck- und Strömungsverhältnissen erfolgt mit Hilfe der wenigstens einen Strömungsmaschine vorzugsweise unter Maßgabe einer funktions- und energieoptimierten Aufrechterhaltung der Barrierewirkung durch die Schutzhülle.

Die Schutzhülle ist ausschließlich aus reinraumtauglichen Materialien gefertigt, wodurch sichergestellt ist, dass von Seiten der Schutzhülle keinerlei Kontaminationen in einen Reinraumbereich gelangen können.

Die lösungsgemäße Vorrichtung eignet sich insbesondere zur Schaffung und/oder Aufrechterhaltung eines reinheitstechnisch kontrollierten Raumbereiches innerhalb des von der Schutzhülle separierten Raumbereiches.

Insbesondere eignet sich die lösungsgemäße Vorrichtung als Einrichtung zur Schaffung eines viren- und querkontaminationsfreien Raumbereiches, der für eine sichere Unterbringung quarantänepflichtiger Personen und/oder Objekte geeignet ist. Hierzu ist die Schutzhülle kuppelzelt- oder kammerartig ausgebildet und grenzt an einen Bodenbereich unmittelbar an, um zusammen mit dem Bodenbereich den Raumbereich zu begrenzen. Die Schutzhülle weist wenigstens zwei, eine erste und eine zweite Schicht auf, die durch eine Abstandsschicht getrennt sind, wobei wenigstens die erste Schicht strömungsdurchlässig ist und die Abstandsschicht eine die beiden Schichten auf Abstand haltende und in Schichtlängserstreckung durchströmbare Tragstruktur besitzt.

Wenigstens eine, vorzugsweise als Filter-Ventilator-Einheit ausgebildete Strömungseinheit ist fluidisch mit der Abstandsschicht verbunden und speist die Abstandsschicht mit einer Luftströmung, vorzugsweise in Form von Reinstluft oder zumindest mit einer viren- und biokontaminationsfreien Zuluft. Die in die Abstandsschicht eingespeiste viren- und biokontaminationsfreie Zuluft gelangt aufgrund der strömungsdurchlässigen, dem Raumbereich zugewandten ersten Schicht in den von der Schutzhülle begrenzten Raumbereich. Vorzugsweise ist auch die der Umgebung zugewandte zweite Schicht strömungsdurchlässig ausgebildet, wodurch von deren Strömungsdurchlässigkeit abhängige Strömungsanteile aus der Abstandsschicht direkt in die Umgebung gelangen.

Die Schutzhülle verfügt zudem über einen Auslassbereich, durch den eine Luftströmung aus dem von der Schutzhülle begrenzten Raumbereich in die Umgebung abgeführt wird. Erfindungsgemäß ist am Auslassbereich eine Sterilisationseinheit angeordnet, die dafür sorgt, dass in die Umgebung durch den Auslassbereich austretende Raumluft vollständig sterilisiert wird.

Die wenigstens eine Strömungseinheit ist erfindungsgemäß derart betreibbar, dass ein sich innerhalb der Abstandsschicht ausbildender Schichtdruck p2 sowohl größer als ein sich innerhalb des Raumbereiches ausbildender Rauminnendruck p1 sowie größer als ein in der Umgebung vorherrschender Umgebungsdruck p3 ist. Auf diese Weise ist sichergestellt, dass der von der Schutzhülle umgebende Raumbereich kontrolliert mit steriler, d.h. Viren- und biokontaminationsfreier Luft durchströmt bzw. durchspült wird und zudem gelangen keine Luftbestandteile außer der durch die vorzugsweise als Filter-Ventilator-Einheit ausgebildete Strömungseinheit in die Abstandsschicht eingespeiste viren- und biokontaminationsfreien Zuluft in den Raumbereich.

Zum Zwecke der Sterilisation von Zu- und Abluft weist die wenigstens eine Strömungseinheit eine Sterilisationseinheit auf. Bei Verwendung einer Sterilisationseinheit ist die Ausbildung der Strömungseinheit als Filter-Ventilator-Einheit nicht notwendigerweise erforderlich, weshalb eine einfache Ventilator-Einheit ausreicht. Längs des Strömungsweges zwischen dem Strömungsausgang der Filter-Ventilator-Einheit bzw. Ventilator-Einheit und der Abstandsschicht sowie längs des Strömungsweges zwischen dem Auslassbereich und dem Strömungseingang der Filter-Ventilator-Einheit bzw. Ventilator-Einheit oder der weiteren Filter-Ventilator-Einheit bzw. weiteren Ventilator-Einheit jeweils eine Sterilisationseinheit zur Herstellung einer Viren- und Biokontaminationsfreien Luftströmung angeordnet sein. Vorzugsweise weist die Sterilisationseinheit eine UVC-Lichtquelle auf, die UV-Licht mit Wellenlängen im Bereich von 220 nm und 270 nm, vorzugsweise 222 nm bzw. 254 nm, zu emittieren vermag.

Die lösungsgemäße Vorrichtung zur Schaffung eines viren- und querkontaminationsfreien Raumbereiches vermag neben der erläuterten Barrierewirkung auch eine Reinraumwirkung zu entfalten. Je nach einstellbarem Luftdurchsatz durch die Abstandsschicht der Schutzhülle und dem einstellbaren, strömungstechnischen Versperrungsgrad der inneren ersten und der äußeren zweiten Schicht kann die Reinraumwirkung verringert oder vergrößert werden. Dadurch ist es möglich Quarantäneschutzräume aufzubauen, die sich nahezu ausschließlich auf die Barrierewirkung beschränken, oder die zusätzlich eine sehr hohe reinheitstechnische Versorgung des Innenbereichs gewährleisten. Dies kann stufenlos variiert werden.

In einer weiteren bevorzugten Ausführungsform sind zur Feststellung von Betriebszuständen als auch zum Regeln und Steuern des reinheitstechnischen Quarantänesystems Sensoren in oder an der Schutzhülle bzw. in der wenigstens einen Strömungseinheit implementiert. Diese Sensoren vermögen vorzugsweise Biokontaminationen, Viren, Partikel oder auch z.B. Druck-/Temperatur-/Feuchtigkeit detektieren.

Auf die weiteren bevorzugten Betriebsweisen der lösungsgemäß ausgebildeten Schutzhülle wird auf die figürlich dargestellten Ausführungsbeispiele im Weiteren verwiesen.

### Kurze Beschreibung der Erfindung

Die Erfindung wird nachstehend ohne Beschränkung des allgemeinen Erfindungsgedankens anhand von Ausführungsbeispielen unter Bezugnahme auf die Zeichnungen exemplarisch beschrieben. Es zeigen:
- Fig. 1: schematisierte Darstellung einer nicht unter die Erfindung fallenden Schutzhülle sowie Illustration des Wirkprinzips (nicht Gegenstand von Anspruch 1),
- Fig. 2: schematisierte Darstellung eines Ausführungsbeispiels zur Realisierung der nicht unter die Erfindung fallenden Schutzhülle mit vier Schichten und zwei Abstandsschichten (nicht Gegenstand von Anspruch 1),
- Fig. 3: schematisiert dargestellte, nicht unter die Erfindung fallende Schutzhülle mit Sensorik (nicht Gegenstand von Anspruch 1),
- Fig. 4: nicht unter die Erfindung fallende Systemanordnung mit Schutzhülle zum Betrieb einer produktionstechnischen Einheit (nicht Gegenstand von Anspruch 1),
- Fig. 5: nicht unter die Erfindung fallende Anordnung einer Quarantäne-Kammer mit einer Filter-Ventilator-Einheit (nicht Gegenstand von Anspruch 1)
- Fig. 6: nicht unter die Erfindung fallende schematisierte Darstellung einer beispielhaft ausgebildeten Schutzhülle für eine Quarantäne-Kammer sowie Illustration des Wirkprinzips (nicht Gegenstand von Anspruch 1),
- Fig. 7: nicht unter die Erfindung fallende Anordnung einer Quarantäne-Kammer mit zwei Filter-Ventilator-Einheiten (nicht Gegenstand von Anspruch 1),
- Fig. 8: Erfindungsgemäße Ausführungsform einer Vorrichtung mit einer Schutzhülle sowie
- Fig. 9: Erfindungsgemäße Ausführungsform der Vorrichtung mit einer Schutzhülle als Mehrfach-Modulanordnung mit einer Vielzahl einzelner Quarantäne-Kammern.

### Nicht unter die Erfindung fallende Beispiele von Schutzhüllen und deren Verwendung

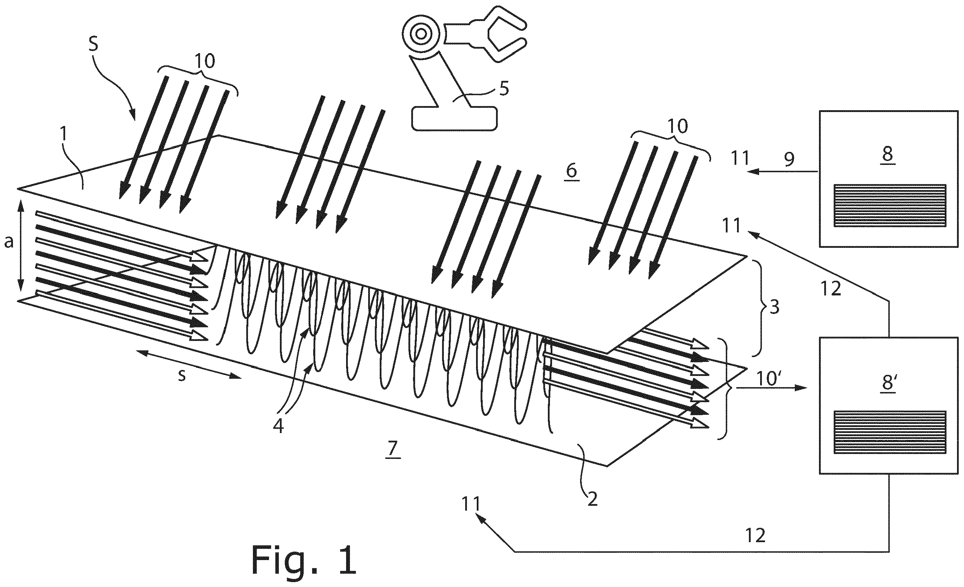

Figur 1 zeigt eine nicht unter die Erfindung fallende Schutzhülle S mit einer ersten Schicht 1, einer zweiten Schicht 2 sowie einer Abstandsschicht 3, die die erste und zweite Schicht 1, 2 mit einem Abstand a zueinander lagert. Die erste und zweite Schicht 1, 2 bestehen vorzugsweise aus einem reinheitstechnischen Textilmaterial mit einer herstellungsbedingten Strömungsdurchlässigkeit durch das Textilgewebe hindurch. Die erste Schicht 1 verfügt über eine Strömungsdurchlässigkeit, vorzugsweise in einem Bereich zwischen 500 und 49000 m³/m²/h, wohingegen die zweite Schicht 2 strömungsundurchlässig gestaltet ist, d. h. über eine Strömungsdurchlässigkeit im Bereich von 0 und 100 m³/m²/h aufweist. Zur mechanischen Beabstandung der ersten und zweiten Schicht 1, 2 weist die Abstandsschicht 3 eine innere Tragstruktur 4, vorzugsweise in Form eines Abstandsgewirkes auf, das in Schichtlängserstreckung s über eine nahezu ungehinderte Durchströmbarkeit verfügt.

Alternativ zur Ausbildung der zweiten Schicht 2 durch Verwendung eines reinheitstechnischen Textilmaterials ist es ebenso denkbar, alternatives, reinheitstechnisches Material einzusetzen, wie bspw. in Form einer Kunststofffolie.

Die Schutzhülle S ist mit ihrer strömungsdurchlässigen ersten Schicht 1 der produktionstechnischen Einheit 5 zugewandt orientiert angeordnet und mit dieser, vorzugsweise raumfest verbunden, so dass die Schutzhülle S einen die Einheit 5 enthaltenden Raumbereich 6 gegenüber der die Schutzhülle S umgebenden Umgebung 7, die dem Reinraum entspricht, separiert, d.h. fluiddicht bzw. kontaminationsfrei abdichtet. Die Anbringung der Schutzschicht S an der produktionstechnischen Einheit 5 bzw. an Teilbereichen der produktionstechnischen Einheit 5 erfolgt mit an sich bekannten Befestigungsmitteln, die nicht Gegenstand dieser Erfindung sind und dem Fachmann bekannt sind.

Zudem ist eine Strömungseinheit 8 vorgesehen, vorzugsweise in Form einer Filter-Ventilator-Einheit, die Reinstluft 11 aus Umgebungsluft herzustellen vermag, die über eine Fluidleitung 9 in den Raumbereich 6 eingespeist wird. Die Einspeisung der Reinstluft 11 in den Raumbereich 6 führt zu einem Druckunterschied zum Inneren der Abstandsschicht 3, so dass sich eine Stoffströmung 10 aus dem Raumbereich 6 durch die erste Schicht 1 in die Abstandsschicht 3 ausbildet.

Die Menge der durch die erste Schicht 1 hindurchtretenden Stoffströmung 10 ist vom Druckunterschied zwischen Raumbereich 6 und dem Inneren der Abstandsschicht 3 sowie durch die Wahl der Strömungsdurchlässigkeit der ersten Schicht 1 abhängig.

Die durch die erste Schicht 1 hindurchtretende Stoffströmung 10 umfasst zum einen die zugeführte Reinstluft, die bedingt durch den Betrieb der produktionstechnischen Einheit innerhalb des Raumbereiches 6 mit Partikeln o.ä. kontaminiert ist. Neben Partikelkontaminationen kann die Stoffströmung auch durch chemische, biologische oder ähnliche Kontaminationen verunreinigt sein, die allesamt durch die erste Schicht 1 hindurchtreten und innerhalb der Abstandsschicht 3 zur weiteren Ableitung der Stoffströmung 10` abströmen. Da die zweite Schicht 2 eine Barriere gegenüber der innerhalb der Abstandsschicht 3 eintretenden Stoffströmung 10 darstellt, wird die dem Reinraum entsprechende Umgebung 7 weder verunreinigt noch kontaminiert. Die längs der Schichtlängserstreckung s abströmende Stoffströmung 10' kann drucklos über eine nicht in Figur 1 dargestellte Entsorgungsleitung entsorgt werden.

In einer weiteren nicht unter die Erfindung fallenden Ausführungsform ist eine weitere Strömungseinheit 8' vorgesehen die als Saugeinheit dient und fluidisch mit der Abstandsschicht 3 verbunden ist. Über die als Saugeinheit arbeitende Strömungseinheit 8' wird die Stoffströmung 10' mittels vorgebbarer Saugleistung kontrolliert aus der Abstandsschicht 3 abgeführt. In vorteilhafter Weise vermag die gleichfalls als Filter-Ventilator-Einheit ausgebildete Strömungseinheit 8' die kontaminierte Stoffströmung 10' durch entsprechende Filterung in Reinstluft 11 umzuwandeln, die über eine Ableitung 12 der Umgebung 7, d. h. dem Reinraum, oder dem Raumbereich 6 wieder zugeführt wird.

Neben der Schutzfunktion der Schutzhülle S vermag die Schutzhülle S ebenso zur Entwärmung bzw. Kühlung der in Betrieb befindlichen produktionstechnischen Einheit 5 dienen, zumal die in den Raumbereich 6 zugeführte Reinstluft zudem auch Wärme durch die erste Schicht 1 und durch Abströmen längs der Abstandsschicht 3 abzuführen vermag.

Alternativ zur Einspeisung von Reinstluft 11 in den Raumbereich 6 eignet sich die Einspeisung eines technischen Gases, bspw. H₂O₂, mittels der Strömungseinheit 8, die in diesem Fall mit einem entsprechenden technischen Gasreservoir, nicht dargestellt, verbunden ist.

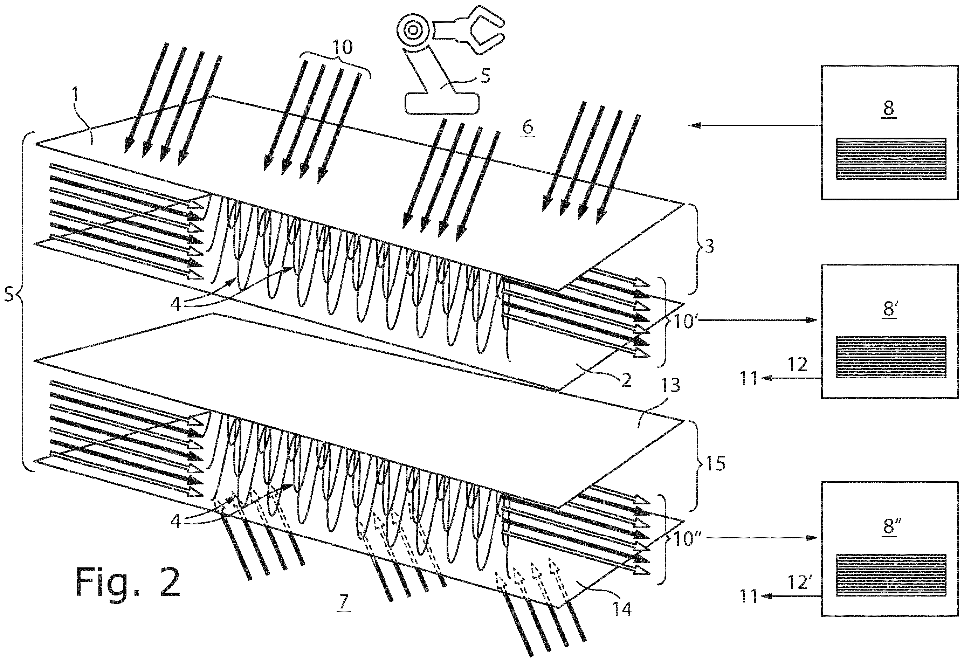

Figur 2 zeigt ein nicht unter die Erfindung fallendes erweitertes Ausführungsbeispiel einer Schutzhülle S, die aus einer Doppelung der in Figur 1 dargestellten Schichtanordnung besteht. So weist die Schutzhülle S gemäß Figur 2 eine erste Schicht 1, eine zweite Schicht 2, eine dritte Schicht 13 sowie eine vierte Schicht 14 auf. Nur aus Gründen einer besseren Illustration ist die zweite Schicht 2 sowie auch die dritte Schicht 13 voneinander beabstandet dargestellt, die ansonsten zu einer einheitlichen Schicht gefügt oder einstückig, d. h. monolithisch ausgebildet sind. Die erste Schicht 1 sowie auch die vierte Schicht 14 bestehen aus einem strömungsdurchlässigen textilen Reinraummaterial jeweils mit einer Strömungsdurchlässigkeit zwischen 500 und 49000 m³/m²/h. Die zweite Schicht 2 sowie dritte Schicht 13 sind aus strömungsundurchlässigem Textilmaterial gefertigt. Die zwischen der dritten Schicht 13 und vierten Schicht 14 eingeschlossene, weitere Abstandsschicht 15 besitzt in gleicher Weise wie die Abstandsschicht 3 eine Tragstruktur 4, durch die die dritte und vierte Schicht 13, 14 auf Abstand gehalten werden.

Gleichsam der Darstellung in Figur 1 ist die Schutzhülle S mit ihrer ersten Schicht 1 der produktionstechnischen Einheit 5 zugewandt orientiert, wohingegen die vierte Schicht 14 der Umgebung 7, die dem Reinraum entspricht, zugewandt orientiert ist. Um Wiederholungen zu vermeiden seien auf die vorstehenden Erläuterungen zur Funktionsweise der Strömungseinheiten 8 und 8' in Bezug auf die Erzeugung und Einspeisung von Reinstluft 11 in den Raumbereich 6 bzw. der kontrollierten unterdruckgetriebenen Abführung der kontaminierten Stoffströmung 10' mit Hilfe der Strömungseinheit 8' und der optionalen Aufbereitung zu Reinstluft 11 verwiesen.

Mit der weiteren Abstandsschicht 15, die durch die dritte und vierte Schicht 13, 14 begrenzt wird, ist fluidisch eine weitere Strömungseinheit 8" verbunden, die als Unterdruckquelle betrieben wird und eine Stoffströmung 10" abzusaugen und optional in Reinstluft 11 umzuwandeln vermag, die der Umgebung 7 über eine Ableitung 12' zuführbar ist. Mit Hilfe der als Unterdruckquelle dienenden Strömungseinheit 8" wird Luft L aus der Umgebung 7 durch die vierte Schicht 14 in die weitere Abstandsschicht 15 gesaugt. Auf diese Weise erfährt die Umgebung 7, die dem Reinraum entspricht, eine zusätzliche Reinigungswirkung.

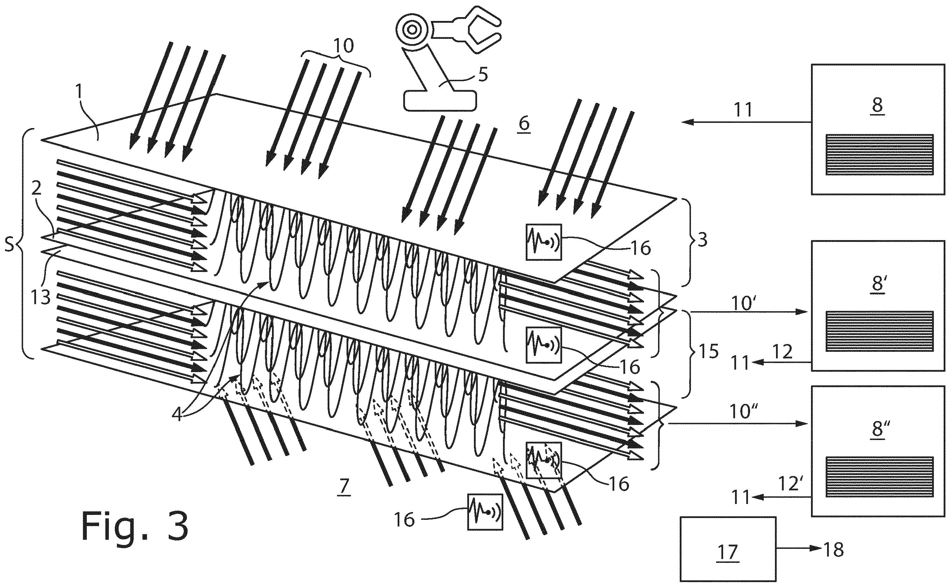

Figur 3 zeigt eine nicht unter die Erfindung fallende Schutzhülle S gemäß der in Figur 2 dargestellten Ausbildungsform die ergänzt ist durch wenigstens einen Sensor 16, der an oder in der Schutzhülle S angebracht ist. Die in Figur 3 illustrierte Anzahl von Sensoren 16 sowie deren Orte zur Anbringung an bzw. in der Schutzhülle S veranschaulichen bevorzugte Möglichkeiten, die den allgemeinen Erfindungsgedanken jedoch nicht beschränken. Die an bzw. in den einzelnen Schichten 1, 2, 3, 13, 14, 15 angebrachten Sensoren 16 können je nach Anforderungen aus den Sensoren der nachfolgenden Art beliebig gewählt werden: Sensor für partikuläre Kontamination (PRC), Sensor für molekulare oder chemische Kontamination (MOC), Temperatursensor (T), Drucksensor (p), Feuchtesensor (h), Strömungssensor, Elektrostatiksensor (ESD), Sensor für Biokontaminationen etc..

Mit Hilfe der an der Schutzhülle S angebrachten Sensoren 16 lassen sich nicht nur Kontaminationskenngrößen erfassen, vielmehr kann der Zustand der Schutzhülle S, die Reinraumqualität der Umgebung 7 sowie auch die Funktionsweise der produktionstechnischen Einheit 5 erfasst und überwacht werden. Die von den einzelnen Sensoren 16 generierten Sensorsignale werden einer Auswerte- und Steuereinheit 17 zugeführt, die wenigstens ein Signal 18 zu generieren vermag, das zu einer Vielzahl weiterer Funktionen nutzbar ist: Zum einen dient das Signal 18 dem kontrollierten Betrieb der einzelnen Strömungseinheiten 8, 8', 8". Ferner kann das Signal 18 der Steuerung der produktionstechnischen Einheit 5 zugrunde gelegt werden. Darüber hinaus kann Signal 18 mittels einer KI-Software o.ä. zur Analyse und Prädiktion der Schutzfunktion und Zuverlässigkeit der in Betrieb befindlichen Schutzhülle dienen.

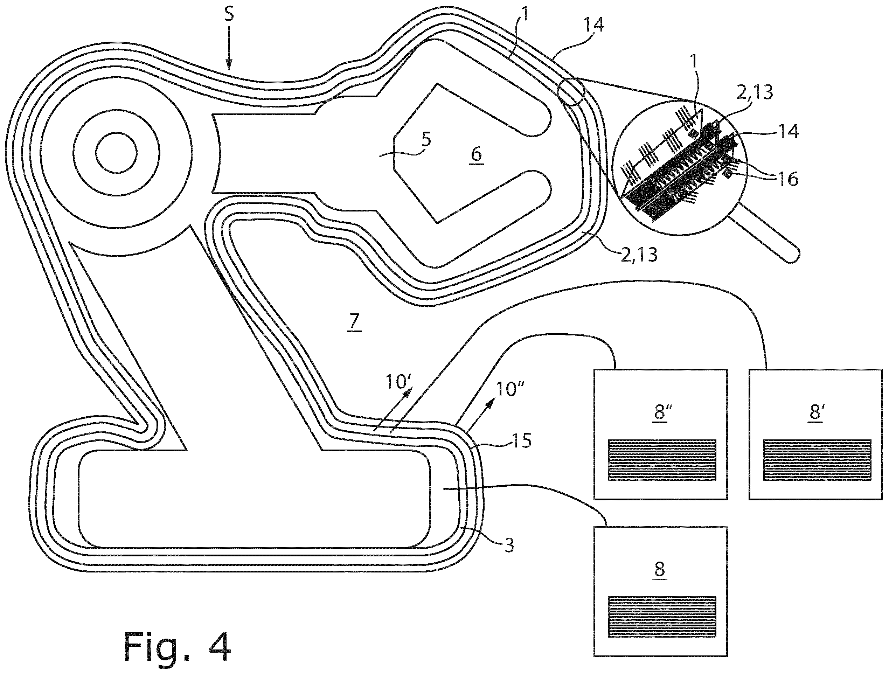

Figur 4 zeigt eine schematisierte Anordnung der nicht unter die Erfindung fallenden Schutzhülle S um eine produktionstechnische Einheit 5 zur Abgrenzung gegenüber der Umgebung 7, die vorzugsweise als Reinraum ausgebildet ist. Es sei angenommen, dass die Schutzhülle S in Art der in Figur 3 illustrierten Ausführungsform ausgebildet ist, siehe Vergrößerungslupe. Die erste Schicht 1 ist der produktionstechnischen Einheit 5 zugewandt orientiert und schließt den Raumbereich 6 ein, wohingegen die vierte Schicht 14 der Umgebung 7, d.h. dem Reinraum zugewandt orientiert ist. Die dazwischen befindlichen Abstandsschichten 3 und 15 sind durch die strömungsundurchlässigen Schichten 2, 13 getrennt.

Zum Betrieb der Schutzhülle S sind drei Strömungseinheiten 8, 8', 8" vorgesehen.

Die Strömungseinheit 8 speist Reinstluft 11 bzw. ein technisches Gas, vorzugsweise H2O2, in den Raumbereich 6 ein und erzeugt hier einen Überdruck gegenüber den in der Umgebung 7 zumeist vorherrschenden atmosphärischen Druckbedingungen. Die Strömungseinheit 8' ist mit der Abstandsschicht 3 fluidisch verbunden und saugt eine kontaminierte Stoffströmung 10' ab. In gleicher Weise vermag die als Saugeinheit dienende Strömungseinheit 8""eine Stoffströmung 10" aus der weiteren Abstandsschicht 15 abzusaugen.

Die in Figur 4 illustrierte Schutzhüllenanordnung S sorgt für eine hohe Kontaminationsbarrieren-Sicherheit und gewährleistet die Reinraumbedingung innerhalb der Umgebung 7 während der in Betrieb befindlichen produktionstechnischen Einheit 5.

Die Schutzhülle S mit den darin integrierten Sensoren 16 werden vorzugsweise um die Einheit 5, z.B. Roboter, montiert, gebracht und/oder übergestülpt, sodass eine strömungstechnische Einhausung um die Einheit 5 entsteht.

Die Vorrichtung führt in Bezug auf die Sauberkeit und Hygiene (Eigenemmissionsarmut der Systemelemente, kontrollierter Innenbereich in Bezug auf partikuläre, chemische und mikrobiologische Kontaminanten) zu folgenden Vorteilen:
Die Strömungseinheiten, in Form von Filter-Ventilator-Einheit, erzeugen einen definierten Volumenstrom zu Reinluftqualitäten der Luftreinheitsklassen 1 bis 9, wie diese in ISO 14644-1 definiert sind.

Varianten zur definierten Strömungsführung zur Gewährleistung eines definierten Über-/Unterdrucks:
- Realisierung durch mindestens einen Reinheitstechnischer Textil- bzw. Mehrschichtbezug, der aus mindestens einem Layer, d.h. erste und zweite Schicht mit der dazwischen befindlichen Abstandsschicht, aufgebaut ist. Die Durchlässigkeitsgrade der einzelnen Schichten kann variabel, entsprechend den einzuhaltenden Sauberkeitsspezifikationen, gewählt werden.
- Die Herstellung der Sauberkeit (Montage, Inbetriebnahme) erfolgt in einem kurzen Zeitfenster (schnelles Auf- und Abbauen).

Geringe Eigensteifigkeit wodurch der Bewegungsablauf nur minimal eingeschränkt wird.
- Die Vorrichtung zur Herstellung der Sauberkeit ist von geringem Gewicht.
- Einsatz unter reinheitstechnisch kontrollierten Bereichen.
- Die Vorrichtung erfüllt die Sauberkeits-/Hygieneanforderungen, wie diese in den relevanten Regelwerkfamilien, wie z.B. der ISO 14644, der VDI 2083, der cGMP Klassen A bis D (beschränkt die Anzahl der Luftkeime) beschrieben sind.
- Die Vorrichtung erfüllt die Möglichkeit der maschinellen Reinigbarkeit der Komponenten.
- Die Vorrichtung erlaubt eine layerspezifische Beaufschlagung mit technischen Gasen, wodurch sowohl ein Schutz der Umgebung, des Produktes als auch der Automatisierungseinheit erreicht werden kann
- Geringe Haftkräfte zwischen Textil- bzw. Mehrschichtbezug und Automatisierungskomponenten
- Die Durchströmbarkeit ist einstellbar, wodurch einzelne Zonen, beispielsweise in Abhängigkeit des Verschmutzungsgrades, individuell ausgelegt und verstärkt mit Reinstluft versorgt werden können. Gleiches gilt für andere Anwendungsbereiche bzw. Gase z.B. zur Abführung von Wärmelasten oder dem Erhalt einer Schutzgasatmosphäre
- Die Messverfahren erlauben eine Vielzahl an Analysen, sowohl über den Hergang, den Zustand der Automatisierungseinheit wie auch über den Zustand des eigentlichen Systems, wodurch sich eine hohe Zuverlässigkeit als auch Systemstabilität erreichen lässt.
- Die Konstruktion des Schutzhüllenmaterials in produktspezifischer Ausführung (mit einstellbarer/konfektionierbarer Permeabilität): von komplett geschlossen (luftdicht) über semipermeabel bis zu strömungstechnisch hoch-geöffnet.

Das Schutzhüllenmaterial, d.h. das Material der ersten bis vierten Schicht kann zusätzlich noch folgende weitere Eigenschaften besitzen:
- elektrostatisch ableitfähiges Hüllenmaterial der Textil- /Mehrschichtbezugs
- partikelarmes Hüllenmaterial
- ausgasungsarmes Hüllenmaterial
- mikrobizides und sterilisierbares Hüllenmaterial
- partikelabsorbierendes bzw. filterndes Hüllenmaterial
- ohne zusätzliche Stützkonstruktion
- komplette Absaugung der Produktionsanlage, ohne zusätzliche Schläuche; quasiintegrale Absaugung
- quasiintegrale Absaugung = Innenseite der Schutzhülle (Richtung Anlagen-/Automatisierungskomponente gewandt) und Aussenseite der Schutzhülle werden gemeinsam ins Innere des Abstandsgewirkes abgesaugt.
- Textil- /Mehrschichtbezug (Abstandsmaterial) fungiert als integrale Absaugung
- innenliegende luftdurchlässige Rotationsachsenkonstruktion für die Unterdruck-Absaugung
- enganliegende Textil- /Mehrschichtbezug, trägt wenig auf und minimale Bewegungseinschränkung
- elektrisch leitend
- brandhemmend.

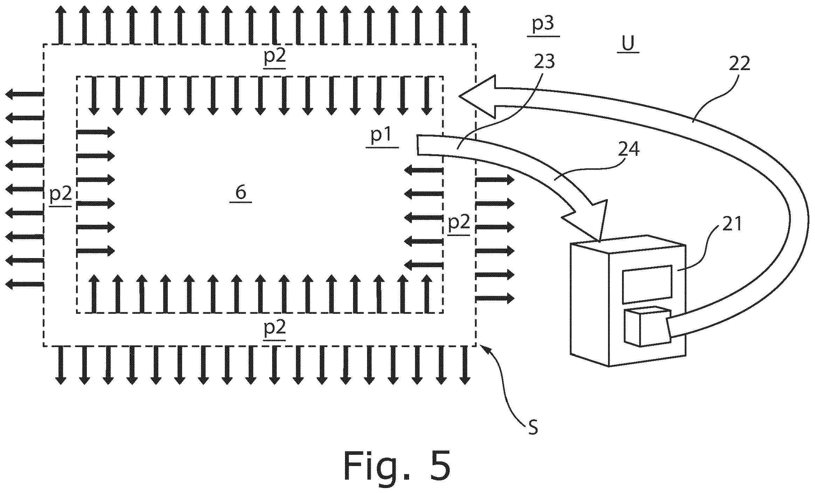

Die vorstehenden Materialien eignen sich ebenso für eine Schutzhülle zur Verwendung und den schnellen Aufbau einer temporären oder dauerhaften Einrichtung zur Schaffung eines viren- und querkontaminationsfreien Raumbereiches 6 zur Unterbringung quarantänepflichtiger Personen und/oder Objekte. Figur 5 zeigt ein strömungstechnisch autarkes Quarantänesystem in einer Querschnittsdarstellung von oben. Hierbei handelt es sich um einen zeltähnlichen Aufbau aus einem textilen Mehrschichtsystem, das im dargestellten Fall als Einpersonen-Quarantänesystem eingesetzt wird.

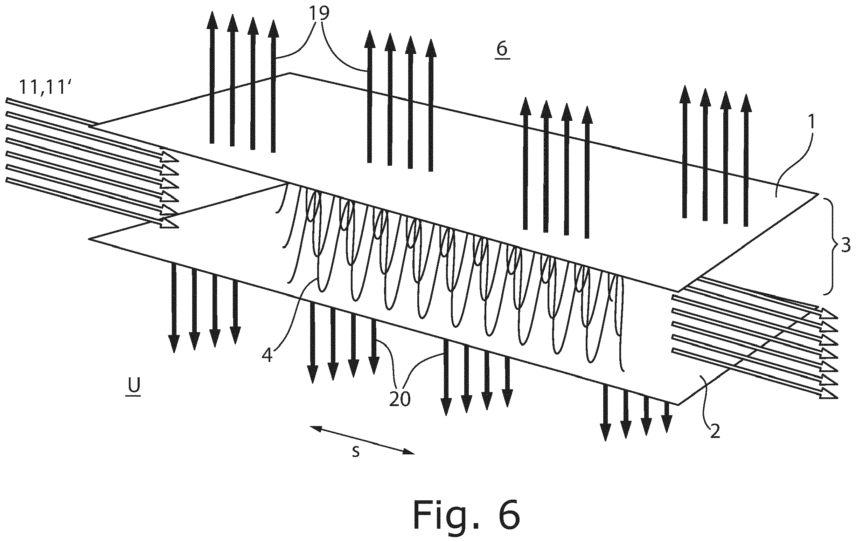

Die den Raumbereich 6 begrenzende Schutzhülle S besteht im gezeigten Ausführungsbeispiel aus einer, dem Raumbereich 6 zugewandten ersten Schicht 1, einer zweiten Schicht 2 und einer beide Schichten 1, 2 voneinander beabstandenden Abstandsschicht 3. Figur 6 zeigt den Schichtaufbau im Detail. Die erste und zweite Schicht 1, 2 bestehen, wie auch im Falle der vorstehend erläuterten Ausführungsbeispiele, vorzugsweise aus einem reinheitstechnischen Textilmaterial mit einer herstellungsbedingten Strömungsdurchlässigkeit durch das Textilgewebe hindurch. Beide Schichten 1 und 2 verfügen über eine Strömungsdurchlässigkeit, vorzugsweise in einem Bereich zwischen 500 und 49000 m³/m²/h. Nicht notwendigerweise sind die Strömungsdurchlässigkeiten beider Schichten nicht identisch zu wählen, sondern bedarfsweise aufeinander abgestimmt zu wählen. Vorzugweise besteht die dem Raumbereich 6 zugewandte erste Schicht 1 aus einem technischen Textilmaterial mit einem geringeren strömungstechnischen Durchlassgrad und die der Umgebung U zugewandte zweite Schicht 2 aus einem technischen Textilmaterial mit einem höheren strömungstechnischen Durchlassgrad.

Zur mechanischen Beabstandung der ersten und zweiten Schicht 1, 2 weist die Abstandsschicht 3 eine innere Tragstruktur 4, vorzugsweise in Form eines Abstandsgewirkes auf, das in Schichtlängserstreckung s über eine nahezu ungehinderte Durchströmbarkeit verfügt.

In die Abstandschicht 3 wird Reinstluft 11 oder zumindest eine Viren- und Biokontaminationsfreie, sterile Luftströmung 11' eingespeist. Durch die kontinuierliche sterile Strömungseinspeisung in die Abstandsschicht 3 bildet sich in Abhängigkeit der gewählten Strömungsdurchlässigkeiten in der ersten und zweiten Schicht 1, 2 sowie den damit verbundenen, kontinuierlich abströmenden Luftströmungsanteilen, die durch die erste Schicht 1 in den Raumbereich 6, siehe Luftströmungsanteil 19, sowie durch die zweite Schicht 2 in die Umgebung U, siehe Luftströmungsanteil 20, gelangen, einen Schichtinnendruck p2 aus. Die Strömungseinspeisung erfolgt vorzugweise derart, dass für den Schichtinnendruck p2, für den sich im Raumbereich 6 ausbildenden Rauminnendruck p1 sowie für den Umgebungsdruck p3, der für gewöhnlich dem atmosphärischen Umgebungsdruck entspricht, folgender Zusammenhang gilt: p2 > p1 und p2 > p3 sowie vorzugsweise p1 > p3.

Für die kontinuierliche Strömungseinspeisung sorgt im Ausführungsbeispiel gemäß Figur 5 eine Strömungseinheit 21, vorzugsweise in Form einer Filter-Ventilator-Einheit, die derart konditioniert ist, Reinstluft oder zumindest sterile Viren- und Biokontaminationsfreie Zuluft druckbeaufschlagt zu erzeugen und diese in die Abstandsschicht 3 über eine sterile Zuleitung 22 einzuspeisen. Die seitens Strömungseinheit 21 herrührende Druckbeaufschlagung der Abstandsschicht 3 der Schutzhülle S sowie auch die Konfektionierung der Schutzhülle S selbst sind vorzugsweise derart gewählt, dass sich die zelt- und/oder kammerähnlichen Quarantäneschutzvorrichtung ausschließlich durch den sich in der Abstandsschicht 3 ausbildenden Überdruck p2 selbsterrichtend und selbsttragend raumstabil ausbildet. Selbstverständlich können bedarfsweise für eine raumstabile Anordnung und Ausbildung der Quarantäneschutzvorrichtung an sich bekannte Trag-, Stütz- und/oder Aufhängevorkehrungen für die Schutzhülle S Verwendung finden. So kann der statisch-mechanische Aufbau und die Steifigkeit der

Quarantäneschutzvorrichtung alternativ durch folgende konstruktive Varianten ausgeführt werden:
- ein tragendes und stützendes außenliegendes Gerüst/Gestänge oder Fachwerkgerüst, an welchem die textile Schutzhülle, oder Teile davon, befestigt wird,
- eine selbsttragende textile Schutzhülle, die in Teilen oder in Gänze ihre Steifigkeit durch den angelegten Innendruck erhält, der oberhalb des umgebenden Umgebungsdruckes liegt,
- eine selbsttragende textile Mehrschichthülle, die in Teilen oder in Gänze eine Eigensteifigkeit aufweist und somit selbsttragend ist.

Um zu gewährleisten, dass die Umgebung U durch die Quarantäneschutzvorrichtung keine Kontaminationen erfährt, weist die Schutzhülle S einen definierten Auslassbereich 23 auf, der mit einer zur Strömungseinheit 21 führenden Ableitung 24 fluidisch verbunden ist. In der Strömungseinheit 21 erfolgt eine Sterilisation der aus dem Raumbereich 6 auströmenden Abluft, die beispielsweise in einem geschlossenen Kreislauf nach erfolgter Sterilisation der Zuleitung 22 wieder zugeführt oder an die Umgebung U abgegeben wird. Je nach Bedingungen vor Ort können auf diese Weise ein vollständig gegenüber der Umgebung U abgeschlossener Betrieb der Quarantäneschutzvorrichtung durch eine "closed-loop-Luftströmung" realisiert oder aber die Umgebungsluft nach vollständiger Sterilisation genutzt werden, wobei nach einer gleichmäßigen Durchströmung des Raumbereiches 6 mit steriler Luft eine Abführung der kontaminierten Luft in die Umgebung U nach entsprechender Sterilisierung erfolgen kann.

Durch den geringen Material- und Komponenteneinsatz lässt sich die Schutzvorrichtung innerhalb weniger Minuten errichten und in Betrieb nehmen.

Das strömungsdurchlässige textile Material zur Realisierung der als Mehrschichtsystem ausgebildeten Schutzhülle kann als flexible, elastische oder auch als starre Schicht ausgeführt werden, bspw. in Form von Gewebeschichten, Folienschichten oder Plattenmaterial mit definierten Perforationsgraden, wodurch eine Bevorratung und Lagerung der Schutzvorrichtungen im nicht Luft-befüllten Zustand auch in großer Stückzahl keine großen Lagerräume erfordert. Das strömungsdurchlässige Material entspricht vorzugsweise jenen Reinheitsanforderungen, denen auch das in den vorstehenden Ausführungsbeispielen in den Figuren 1 bis 4 eingesetzte Textilmaterial entspricht. Für den Aufbau der erläuterten Druckverhältnisse, d.h. p1 <p3<p2, können mindestens eine oder auch mehrere Strömungseinheiten eingesetzt werden, die entweder mit Überdruck oder mit Unterdruck arbeiten, um entweder gezielt kontaminierte Luft aus dem Raumbereich 6 abzusaugen und aufzunehmen, oder umgekehrt, die inneren Kontaminationen gezielt vom Außenbereich abzuschirmen.

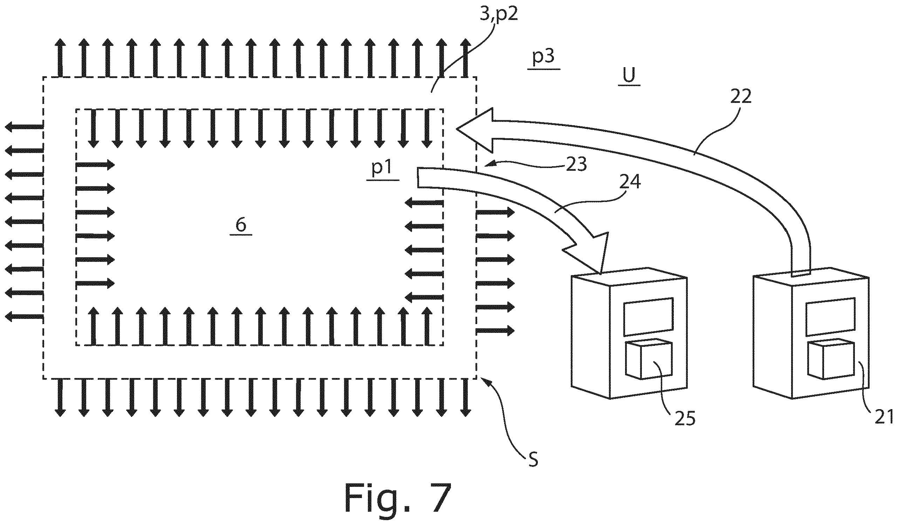

Figur 7 zeigt eine nicht unter die Erfindung fallende Ausführungsvariante, bei der die Abstandsschicht 3 der Schutzhülle S mit einer ersten als Filter-Ventilator-Einheit ausgebildeten Strömungseinheit 21 druckbeaufschlagt mit steriler Zuluft über die Zuleitung 22 gespeist wird. Über eine fluiddicht am Auslassbereich 23 der Schutzhülle S verbundene Ableitung 24 ist eine zweite vorzugsweise als Filter-Ventilator-Einheit ausgebildete Strömungseinheit 25 mit dem Raumbereich 6 verbunden, aus dem die zweite Strömungseinheit 25 kontaminierte Raumluft absaugt und sicher entsorgt, d.h. die abgesaugte kontaminierte Raumluft wird vollständig sterilisiert und an die Umgebung abgegeben.

Zum Zwecke der Sterilisation der Zuluft aus der Umgebung oder der aus dem Raumbereich 6 abgeführten, kontaminierten bietet sich neben bekannten Sterilisationstechniken, wie bspw. Gammasterilisation, H₂O₂-Sterilisation, Schwebstofffilter oder der Wärmeeintrag mittels Heizer, vor allem die Sterilisation mittels UVC-Bestrahlung im Fern-UVC-Wellenlängenbereich bei ca. 222 nm an. Der Einsatz einer UVC-Bestrahlungseinheit kann vielerorts an den Bereichen und Komponenten des Quarantänesystems angebracht werden, bspw. als integrale Strahlungskomponenten innerhalb einer Filter-Ventilator-Einheit, innerhalb des Raumbereiches an der Schutzhülle, an oder innerhalb der Schutzhülle, bspw. in Form einer Vielzahl von LED-Strahler. Insbesondere am Strömungsauslassbereich oder längs der Zu- und Ableitungen können derartige UVC-Bestrahlungseinheiten angeordnet werden.

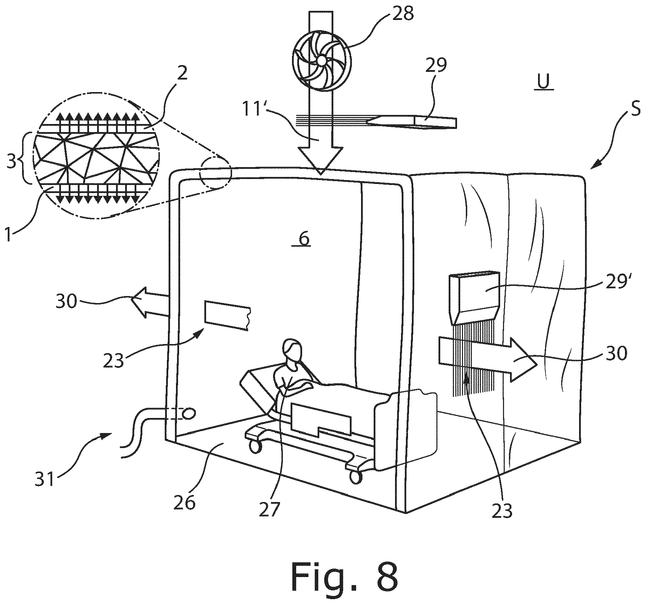

Figur 8 zeigt ein Ausführungsbeispiel der Erfindung in Form einer als Quarantänekammer ausgebildeten Vorrichtung mit einer Schutzhülle S, deren Schutzhülle S zusammen mit einem Bodenbereich 26, an dem die Schutzhülle S umfangsrandbildend, bündig und weitgehend gasdicht aufliegt, einen inneren Raumbereich 6 umschließt, in dem eine quarantänepflichtige Personen 27 untergebracht ist. Denkbar wäre auch eine Quarantänekammer mit einem fest an die Schutzstruktur gefügten, bspw. tuch- oder folienartig ausgebildeten Bodenteil. Über eine schematisch illustrierte Ventilator-Einheit 28 gelangt sterilisierte Zuluft 11' durch die der Umgebung U zugewandte, zweite Schicht 2 in die Abstandsschicht 3 der Schutzhülle S. Eine UVC-Bestrahlungseinheit 29 sorgt für die erforderliche Sterilisation der Zuluft. Überdruckgetrieben tritt kontaminierte Raumluft 30 durch die in der Schutzhülle S vorgesehenen Auslassbereiche 23, an denen jeweils eine weitere UVC-Bestrahlungseinheit 29` zu Sterilisationszwecken angebracht ist, in die Umgebung. Auf diese Weise ist sichergestellt, dass möglichst kein unerwünschter Transport von Viren bzw. Biokontaminationen von der Umgebung zum quarantänisierten Patienten 27 bzw. vom Quarantänepatienten 27 an die umgebende Außenluft abgegeben werden.

Zur Optimierung des Quarantäne-Innenbereichs können auch Zusätze von technischen Gasen 31, z.B. Reinstsauerstoff, zudosiert werden.

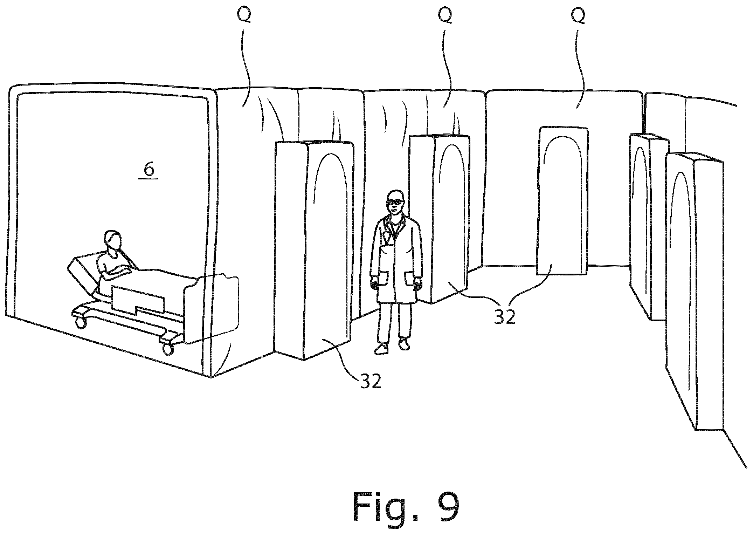

Das in Figur 8 illustrierte Quarantänesystem dient als modulare Baueinheit durch vielfache Zusammenstellung jeweils baugleicher Quarantänemodule Q zur Ausgestaltung und Anordnung eines beliebig groß skalierbaren Quarantäne-Ensembles, siehe Figur 9. Jedes einzelne Quarantänemodule Q besitzt zum kontaminationsfreien Ein- und Austritt eine Zugangsschleuse 32. Ein derartiges Quarantäne-Ensembles kann in beliebigen größeren Hallen errichtet werden, sofern jedem Patienten eine viren- und querkontaminationsfreie Zone zugesichert werden muss. Dies unterbindet zum einen den Ausbreitungsprozess und zum anderen sichert es einen schnelleren Genesungsprozess ohne stetig neu auf den Patienten einwirkende Biokontaminationen.

Zudem kann das Quarantäne-Ensemble durch Zugänge, Durchreichen, Anschlusskorridore etc. mit den im Stand der Technik bekannten Türen, Fenstern, Schleusen, Anschlüssen, Kanälen etc. ergänzt und ausgeführt werden.

Mithin lassen sich die nachfolgenden Vorteile zum erläuterten lösungsgemäßen Quarantänesystem wie folgt benennen:
- Das System lässt sich innerhalb weniger Minuten sehr schnell an belieben Orten weltweit aufbauen und ist in wenigen Minuten nach Inbetriebnahme sofort einsatzfähig.
- Es bietet höchste Sicherheit vor Querkontaminationen und einer zu verhindernden, potenziellen Virenverschleppung
- Das System ist sterilisierbar und waschbar
- Das System ist nach Sterilisation oder Waschen in handlichen Einheiten steril verpackbar, bspw. Einschweißen in dichte/reine Foliensäcke und steht damit sofort nach dem Auspacken für den Einsatz zur Verfügung, kann somit lange steril gelagert werden und ist gut steril transportierbar
- Das System kann im Vorfeld sehr kostengünstig gefertigt werden
- Aufgrund eines sehr kleinen Packmaßes (zusammenfaltbar) müssen keine größeren Platzreserven vorgehalten werden und kann eine platzsparende Einlagerung erfolgen
- Höchster Schutz für Bediener- und Servicepersonal und Ärzte
- Höchster Schutz für die Quarantänepatienten vor Neuinfektion
- Kostengünstiger Betrieb des reinheitstechnischen Quarantänesystems, ohne aufwendige Gas-, Strom-, Chemikalienversorgung
- Die für Patientenversorgung notwendigen Medien (Stromkabel, Datenkabel, Schläuche, etc. ) können sehr schnell und einfach durch eingearbeitete, verschließbare Öffnungen eingebracht werden
- Keine aufwendige Entsorgung von viren-/biokontamionationsbelastetem Material notwendig, bei Bedarf kostengünstige, einfache Entsorgung
- Mehrfachnutzung möglich
- Geringe Anschaffungskosten
- Vernachlässigbare Betriebskosten (nur Strom für Ventilatoreinheit und Strom für den Betrieb der UVC-Sterilisationsleuchten)
- Betriebssichere Anwendung

Bevorzugte Einsatzbereiche für die lösungsgemäße Schutzvorrichtung sind nachfolgend zusammengestellt:
Reinheitstechnisch/hygienisch (frei von Viren/Bakterien und andern Mikroorganismen) kontrollierte Bereiche, wie diese in den folgenden Anwendungsgebieten benötigt werden:
Luft- und Raumfahrt, Optik, Lebenswissenschaften (Biochemie, Bioinformatik, Biologie, Biomedizin, Biophysik, Bio- und Gentechnologie, Impfstoffentwicklung, Medizin (Krankenhaus, Behelfskrankenhaus, Krankenhausbetten, Intensivstationen, OP-Bereiche, Arztpraxen, Stationen in Gesundheitsämtern, Flughäfen, Bahnhöfen und Messehallen, Feldforschung), Medizintechnik, Pharmazie und Pharmakologie, Umweltmanagement und Umwelttechnik), Chemie.

### Bezugszeichenliste

- 1: erste Schicht
- 2: zweite Schicht
- 3: Abstandsschicht
- 4: Tragstruktur/Abstandsgewirk
- 5: produktionstechnische Einheit
- 6: Raumbereich
- 7: Umgebung, Reinraum
- 8, 8', 8": Strömungseinheit
- 9: Fluidleitung
- 10, 10', 10": Stoffströmung
- 11: Reinstluft
- 11': Viren- und Biokontaminationsfreie Luftströmung
- 12, 12': Ableitung
- 13: dritte Schicht
- 14: vierte Schicht
- 15: weitere Abstandsschicht
- 16: Sensor
- 17: Auswerte-/Steuereinheit
- 18: Signal
- 19: durch die erste Schicht in den Raumbereich einströmender Luftanteil
- 20: durch die zweite Schicht in die Umgebung einströmender Luftanteil
- 21: Strömungseinheit, vorzugsweise Filter-Ventilator-Einheit
- 22: sterile Zuleitung
- 23: Auslassbereich
- 24: Ableitung
- 25: zweite als Filter-Ventilator-Einheit ausgebildete Strömungseinheit
- 26: Bodenbereich
- 27: quarantänepflichtige Personen
- 28: Ventilator-Einheit
- 29, 29': UVC-Bestrahlungseinheit
- 30: kontaminierte Raumluft
- 31: Zuführung techn. Gase
- 32: Zugangsschleuse
- S: Schutzhülle
- L: Luft
- U: Umgebung
- Q: Quarantänemodul

## Patentansprüche

1. Vorrichtung mit einer Schutzhülle (S), die dafür geeignet und ausgebildet ist wenigstens einen Raumbereich (6) zu umhüllen, so dass die Schutzhülle (S) den wenigstens einen Raumbereich (6) zelt- oder kammerartig umgibt, an einen Bodenbereich (26) unmittelbar angrenzt und den Raumbereich (6) gegenüber einer die Schutzhülle (S) unmittelbar umgebenden Umgebung (U) zu separieren vermag, wobei die Schutzhülle (S) wenigstens zwei, eine erste und eine zweite, durch eine Abstandsschicht (3) getrennte Schichten (1, 2) aufweist, von denen die Abstandsschicht (3) eine die beiden Schichten (1, 2) auf Abstand haltende und in Schichtlängserstreckung durchströmbare Tragstruktur (4) aufweist und wenigstens eine Strömungseinheit (21) mit einem Strömungsausgang vorgesehen ist, der durch die der Umgebung (U) zugewandte, zweite Schicht (2) der Schutzhülle (S) fluidisch mit der Abstandsschicht (3) verbunden ist,
**dadurch gekennzeichnet, dass** die erste Schicht (1) dem Raumbereich (6) zugewandt und strömungsdurchlässig ist und eine Strömungsdurchlässigkeit im Bereich von 500 bis 49000 m³/m²/h besitzt,
dass zumindest die erste und zweite Schicht (1, 2) ausschließlich aus reinraumtauglichen Material gefertigt sind, das dazu geeignet ist einen Reinraum (6) der ISO-Klassen 1 bis 9 nach DIN EN ISO 14644-1:2016-06 unmittelbar zu begrenzen,
dass die Schutzhülle (S) einen Auslassbereich (23) aufweist, durch den überdruckgetrieben Raumluft in die Umgebung (U) tritt und an dem eine Sterilisationseinheit (29') angebracht ist, und
dass die wenigstens eine Strömungseinheit (21) derart betreibbar ist, dass ein sich innerhalb der Abstandsschicht (3) ausbildender Schichtinnendruck p2 sowohl größer als ein sich innerhalb des Raumbereiches (6) ausbildender Rauminnendruck p1 sowie größer als ein in der Umgebung (U) vorherrschender Umgebungsdruck p3 ist.

2. Vorrichtung nach Anspruch 1,
**dadurch gekennzeichnet, dass** innerhalb der wenigstens einen Strömungseinheit 21) und/oder längs eines Strömungsweges zwischen dem Strömungsausgang der Strömungseinheit (21) und der Abstandsschicht (3) sowie längs eines Strömungsweges zwischen dem Auslassbereich (23) und dem Strömungseingang der Strömungseinheit (28) eine weitere Sterilisationseinheit (29) angeordnet ist.

3. Vorrichtung nach Anspruch 1 oder 2,
**dadurch gekennzeichnet, dass** die Sterilisationseinheit (29, 29') zur Herstellung einer Viren- und Biokontaminationsfreien Luftströmung eine UVC-Lichtquelle besitzt.

4. Vorrichtung nach einem der Ansprüche 1 bis 3,
**dadurch gekennzeichnet, dass** die zweite Schicht (2) eine Strömungsdurchlässigkeit von 0 bis 100 m³/m²/h besitzt.

5. Vorrichtung nach einem der Ansprüche 1 bis 4,
**dadurch gekennzeichnet, dass** eine dritte und eine vierte, durch eine weitere Abstandsschicht (15) getrennte Schichten (13, 14) vorgesehen sind,
dass die dritte Schicht (13) mit der zweiten Schicht (2) über eine Fügung oder einstückig verbunden ist,
dass die vierte Schicht (14) strömungsdurchlässig ist,
dass die weitere Abstandsschicht (15) eine die dritte und vierte Schicht (13, 14) auf Abstand haltende und in Schichtlängserstreckung durchströmbare Tragstruktur (4) besitzt.

6. Vorrichtung nach Anspruch 5,
**dadurch gekennzeichnet, dass** wenigstens eine Strömungseinheit vorgesehen ist, die fluidisch mit der weiteren Abstandsschicht (15) verbunden ist.

7. Vorrichtung nach Anspruch 5 oder 6,
**dadurch gekennzeichnet, dass** die vierte Schicht (14) und die erste Schicht (1) aus einem gleichem Material mit gleichen Materialeigenschaften ausgebildet sind,
dass die dritte Schicht (13) und die zweite Schicht (2) aus einem gleichem Material mit gleichen Materialeigenschaften ausgebildet sind, und
dass die Abstandsschicht (4) und die weitere Abstandsschicht (4) jeweils eine Tragstruktur aus gleichem Material mit gleichen Materialeigenschaften aufweisen.

8. Vorrichtung nach einem der Ansprüche 1 bis 7,
**dadurch gekennzeichnet, dass** die wenigstens eine Strömungseinheit (21, 28) als Überdruckquelle betreibbar ist und einen Strömungseingang aufweist, der mit einem Gasreservoir verbunden ist oder zum Einsaugen von Umgebungsluft in die freie Umgebung mündet.

9. Vorrichtung nach Anspruch 8,
**dadurch gekennzeichnet, dass** die Strömungseinheit (21, 28) als Filter-Ventilator-Einheit zur Herstellung von Reinstluft gemäß DIN EN ISO 14644-1:2016-06 aus Umgebungsluft ausgebildet ist und die fluidische Verbindung zwischen dem Strömungsausgang und der Abstandsschicht (4) als Hohlkanal aus einem reinraumtauglichen Material gemäß DIN EN ISO 14644-14:2017-01 gefertigt ist.

10. Vorrichtung nach einem der Ansprüche 1 bis 9,
**dadurch gekennzeichnet, dass** wenigstens an der ersten Schicht (1), zweiten Schicht (2) und/oder Abstandsschicht (4) wenigstens ein Sensor (16) der nachfolgenden Art angebracht ist:
Sensor für partikuläre Kontaminationen (PAC), Sensor für molekulare oder chemische Kontaminationen (MOC), Temperatursensor (T), Drucksensor (p), Feuchtesensor (h), Strömungssensor, Elektrostatiksensor (ESD), Sensor für Biokontaminationen.

11. Vorrichtung nach Anspruch 10,
**dadurch gekennzeichnet, dass** der wenigstens eine Sensor (16) mit einer Auswerte- und Steuer-, bzw. Regeleinheit (17) verbunden ist, die wenigstens ein Signal generiert, das an eine übergeordnete Software und/oder an die wenigstens eine Strömungseinheit (21, 28) und/oder an die Einheit (17) übertragbar ist.

12. Vorrichtung nach einem der Ansprüche 1 bis 11,
**dadurch gekennzeichnet, dass** wenigstens die Abstandsschicht (4) wenigstens zwei Schichtbereiche aufweist, in denen sich die Durchströmbarkeit in Schichtlängserstreckung voneinander unterscheiden.

13. Verwendung der Vorrichtung nach einem der Ansprüche 1 bis 12 als temporäre oder dauerhafte Einrichtung zur Schaffung eines viren- und querkontaminationsfreien Raumbereiches zur Unterbringung quarantänepflichtiger Personen (27) und/oder Objekte.

## Claims

1. Device with a protective cover (S) which is suitable and designed to cover at least one room region (6) in such a way that the protective cover (S) surrounds the at least one room region (6) in the manner of a tent or chamber, directly adjoins a floor region (26) and is able to separate the room region (6) from an environment (U) immediately surrounding the protective cover (S),
wherein the protective cover (S) includes at least two layers (1, 2), a first and a second layer separated by a spacer layer (3), of which the spacer layer (3) has a support structure (4) that keeps the two layers (1, 2) apart and is flow-permeable in the longitudinal extension of the layers, and at least one flow unit (21) with a flow outlet is provided, which is fluidically connected to the spacer layer (3) via the second layer (2) of the protective cover (S), which is closest to the environment (U),
**characterized in that**
the first layer (1) is mounted closest to the room region (6) and is flow-permeable and has a permeability to flow in the range from 500 to 49,000 m³/m²/h,
at least the first and second layers (1, 2) are made exclusively from clean-room compatible material which is designed to directly enclose a cleanroom (6) of ISO classes 1 to 9 according to DIN EN ISO 14644-1:2016-06,
the protective cover (S) has an outlet area (23), through which room air driven by overpressure exits into the environment (U) and on which a sterilisation unit (29') is mounted; and
the at least one flow unit (21) is operable in such manner that a layer internal pressure p2 created inside the spacer layer (3) is both greater than a room inner pressure p1 formed inside the room region (6) and greater than an ambient pressure p3 prevailing in the surrounding environment (U).

2. Device according to Claim 1, **characterized in that** a further sterilisation unit (29) is arranged inside the at least one flow unit (21) and/or along a flow path between the flow outlet of the flow unit (21) and the spacer layer (3) as well as along a flow path between the outlet area (23) and the flow inlet of the flow unit (28).

3. Device according to Claim 1 or 2, **characterized in that** the sterilisation unit (29, 29') is equipped with a UVC light source for creating a virus-free and biocontamination-free air flow.

4. Device according to any one of Claims 1 to 3, **characterized in that** the second layer (2) has a flow permeability from 0 to 100 m³/m²/h.

5. Device according to any one of Claims 1 to 4,
**characterized in that** a third and a fourth layer (13, 14) are provided, separated by a further spacer layer (15),
the third layer (13) is connected to the second layer (2) either via a joint or integrally therewith,
the fourth layer (14) is flow-permeable,
the further spacer layer (15) has a support structure (4) which keeps the third and fourth layers (13, 14) and is flow-permeable in the longitudinal direction of the layer.

6. Device according to Claim 5, **characterized in that** at least one flow unit is provided which is fluidically connected to the further spacer layer (15).

7. Device according to Claim 5 or 6,
**characterized in that** the fourth layer (14) and the first layer (1) are formed from the same material with the same material properties,
the third layer (13) and the second layer (2) are formed from the same material with the same material properties, and
the spacer layer (4) and the further spacer layer (4) each have a support structure made of the same material with the same material properties.

8. Device according to any one of Claims 1 to 7, **characterized in that** the at least one flow unit (21, 28) is operable as an overpressure source and has a flow inlet that is connected to a gas reservoir or has an opening in the surrounding environment in order to suck in ambient air.

9. Device according to Claim 8, **characterized in that** the flow unit (21, 28) is embodied as a filter-fan unit for producing ultrapure air according to DIN EN ISO 14644-1:2016-06 from ambient air, and the fluidic connection between the flow outlet and the spacer layer (4) constructed in the form of a hollow channel made of a cleanroom-compatible material according to DIN EN ISO 14644-14:2017-01.

10. Device according to any one of Claims 1 to 9,
**characterized in that** at least one sensor (16) of the following type is attached at least to the first layer (1), the second layer (2) and/or the spacer layer (4):
sensor for particulate contamination (PAC), sensor for molecular or chemical contaminations (MOC), temperature sensor (T), pressure sensor (p), humidity sensor (h), flow sensor, electrostatic sensor (ESD), sensor for biocontamination.

11. Device according to Claim 10, **characterized in that** the at least one sensor (16) is connected to an evaluation and control unit (17) that generates at least one signal which can be transmitted to a higher-level software and/or to the at least one flow unit (21, 28) and/or to the unit (17).

12. Device according to any one of Claims 1 to 11, **characterized in that** at least the spacer layer (4) has at least two layer areas in which the flow permeabilities in the longitudinal extension of the layers differ from one another.

13. User of the device according to any one of Claims 1 to 12 as a temporary or permanent facility for creating a virus-free and cross-contamination free room region for accommodating individuals (27) and/or objects that must be quarantined.

## Revendications

1. Dispositif avec une gaine protectrice (S), qui est adaptée et constituée pour envelopper au moins une zone d'espace (6) de telle manière que la gaine protectrice (S) entoure au moins une zone d'espace (6) à la manière d'une tente ou d'une chambre, vient directement jouxter une zone de fond (26) et est capable de séparer la zone d'espace (6) par rapport à un environnement (U) entourant directement la gaine protectrice (S),
sachant que la gaine protectrice (S) comporte au moins deux couches (1, 2), une première et une deuxième, séparées par une couche d'espacement (3), la couche d'espacement (3) comportant une structure porteuse (4) maintenant à distance les deux couches (1, 2) et pouvant être traversée dans l'extension longitudinale de couche et au moins une unité d'écoulement (21) avec une sortie d'écoulement est prévue, qui est reliée fluidiquement à la couche d'espacement (3) par la deuxième couche (2) tournée vers l'environnement (U) de la gaine protectrice (S)
**caractérisé en ce que**
la première couche (1) est tournée vers la zone d'espace (6) et laisse passer un écoulement et possède une capacité à laisser passer un écoulement se situant dans une plage de 500 à 49000 m³/m²/h,
**en ce qu'**au moins la première et la deuxième couche (1, 2) sont fabriquées exclusivement dans un matériau adapté aux salles blanches, qui est adapté pour délimiter directement une salle blanche (6) de catégorie ISO 1 à 9 conformément à la norme DIN EN ISO 14644-1:2016-06,
**en ce que** la gaine protectrice (S) comporte une zone d'évacuation (23) à travers laquelle de l'air ambiant entraîné par surpression pénètre dans l'environnement (U) et sur laquelle est placée une unité de stérilisation (29'), et
**en ce qu'**au moins une unité d'écoulement (21) peut être utilisée de telle manière qu'une pression intérieure de couche p2 se constituant à l'intérieur de la couche d'espacement (3) est aussi grande qu'une pression intérieur d'espace p1 se constituant à l'intérieur de la zone d'espace (6) et aussi grande qu'une pression ambiante p3 régnant dans l'environnement (U).

2. Dispositif selon la revendication 1, **caractérisé en ce qu'**une autre unité de stérilisation (29) est disposée à l'intérieur d'au moins une unité d'écoulement (21) et/ou le long d'une voie d'écoulement entre la sortie d'écoulement de l'unité d'écoulement (21) et de la couche d'espacement (3) ainsi que le long d'une voie d'écoulement entre la zone de d'évacuation (23) et l'entrée d'écoulement de l'unité d'écoulement (28).

3. Dispositif selon la revendication 1 ou 2, **caractérisé en ce que** l'unité de stérilisation (29, 29') possède une source lumineuse UV-C pour réaliser un écoulement d'air exempt de contamination virale et biologique.

4. Dispositif selon l'une quelconque des revendications 1 à 3, **caractérisé en ce que** la deuxième couche (2) possède une capacité à laisser passer un écoulement de 0 à 100 m³/m²/h.

5. Dispositif selon l'une quelconque des revendications 1 à 4,
**caractérisé en ce qu'**une troisième et une quatrième couche (13, 14) séparées par une autre couche d'espacement (15), sont prévues,
**en ce que** la troisième couche (13) est reliée par un assemblage ou en une seule pièce à la deuxième couche (2),
**en ce que** la quatrième couche (14) laisse passer un écoulement
**en ce que** l'autre couche d'espacement (15) possède une structure porteuse (4) maintenant à distance la troisième et la quatrième couche (13, 14) et peut être traversée dans l'extension longitudinale de couche.

6. Dispositif selon la revendication 5, **caractérisé en ce qu'**au moins une unité d'écoulement est prévue, qui est reliée fluidiquement à l'autre couche d'espacement (15).

7. Dispositif selon la revendication 5 ou 6,
**caractérisé en ce que** la quatrième couche (14) et la première couche (1) sont constituées dans le même matériau avec les mêmes propriétés de matériau,
**en ce que** la troisième couche (13) et la deuxième couche (2) sont constituées dans le même matériau avec les mêmes propriétés de matériau, et
**en ce que** la couche d'espacement (4) et l'autre couche d'espacement (4) comportent respectivement une structure porteuse constituée dans le même matériau avec les mêmes propriétés de matériau.

8. Dispositif selon l'une quelconque des revendications 1 à 7, **caractérisé en ce qu'**au moins une unité d'écoulement (21, 28) peut être utilisée sous la forme d'une source de surpression et comporte une entrée d'écoulement, qui est reliée à un réservoir de gaz ou débouche à l'air libre ambiant pour aspirer de l'air ambiant.

9. Dispositif selon la revendication 8, **caractérisé en ce que** l'unité d'écoulement (21, 28) est constituée sous la forme d'une unité ventilateur-filtre pour la fabrication d'air pur conformément à la norme DIN EN ISO 14644-1:2016-06 à partir de l'air ambiant et la liaison fluidique entre la sortie d'écoulement et la couche d'espacement (4) sous la forme d'un conduit creux dans un matériau adapté aux salles blanches conformément à la norme DIN EN ISO 14644-14:2017-01.

10. Dispositif selon l'une quelconque des revendications 1 à 9,
**caractérisé en ce qu'**au moins un capteur (16) de type décrit ci-après est placé au moins sur la première couche (1), la deuxième couche (2) et/ou la couche d'espacement (4) :
capteur pour les contaminations particulières (PAC), capteur pour les contaminations moléculaires ou chimiques (MOC), capteur de température (T), capteur de pression (p), capteur d'humidité (h), capteur de débit, capteur électrostatique (ESD), capteur pour les contaminations biologiques.

11. Dispositif selon la revendication 10, **caractérisé en ce qu'**au moins un capteur (16) est relié à une unité d'évaluation et de commande ou de régulation (17), qui génère au moins un signal, qui peut être transmis à un logiciel subordonné et/ou à au moins une unité d'écoulement (21, 28) et/ou à l'unité (17).

12. Dispositif selon l'une quelconque des revendications 1 à 11, **caractérisé en ce qu'**au moins la couche d'espacement (4) comporte au moins deux zones de couche se distinguant l'une de l'autre par la traversabilité dans l'extension longitudinale de couche.

13. Utilisation du dispositif selon l'une quelconque des revendications 1 à 12 en tant qu'installation temporaire ou permanente destinée à créer une zone d'espace exempte de contamination virale et croisée pour loger des personnes (27) et/ou des objets soumis à quarantaine.
